# EUROPEAN PATENT APPLICATION

(11) **EP 0 858 809 A1**
(43) Date of publication of application: **19.08.1998**
(21) Application number: 96906011.0
(22) Date of filing: 14.03.1996
(51) Int. Cl.: A61K 38/18, A61K 38/19, A61K 47/02, A61K 47/12, A61K 47/14, A61K 47/18, A61K 47/26, A61K 47/32, A61K 47/34, A61K 47/38, A61K 47/42

(54) **METHOD OF PREVENTING TPO ADSORPTION AND STABLE TPO-CONTAINING COMPOSITION**

(30) Priority: 15.03.1995 JP 56248/95
(71) Applicant: Kirin Brewery Company, Ltd., Tokyo 104 (JP)
(72) Inventor: OTSUKI, Naoki, Kirin Brewery Company, Limited, Takasaki-shi, Gunma-ken 370-12 (JP)
(74) Representative: Voelker, Ingeborg Carla Emmy
(86) International application number: JP9600635
(87) International publication number: WO9628181

(57) **Abstract**

A TPO-containing composition comprising a TPO protein having a sugar-chain moiety and at least one pharmaceutically acceptable additive selected from the group consisting of proteins, cellulose derivatives, surface active agents and polyvinyl alcohol; and a method of preventing TPO from being adsorbed on a container wall by using the above composition containing such additives, thereby preventing the titer-reduction caused by the adsorption.

## Description

### BACKGROUND OF THE INVENTION

### 1. Field of the Invention

This invention relates to a composition which contains a TPO protein having a sugar chain moiety, more particularly, to a stable TPO-containing composition which is effective in preventing the loss or titer reduction of the TPO as active component caused by its adsorption on the wall of a container, or by its association, polymerization or the like. This invention also relates to a method for the prevention of TPO titer reduction caused by adsorption of TPO on the wall of a container charged with the composition.

### 2. Disclosure of Related Art

Human TPO (thrombopoietin) is a protein cloned as ligand of Mpl which is a member of the cytokine receptor superfamily (de Sauvage et al., Nature (London), vol.369, pp.533-565 (1994); Bartley, T.D. et al., Cell, vol.77, pp.1117-1124 (1994)). The Mpl ligands can be detected in sera and plasma of thrombocytopenic animals (e.g., human, mouse, dog, etc.), and its involvement in the production of megakaryocytes and platelets has been already confirmed.

To develop a therapeutic agent for use in the treatment of thrombocytopenia, the present inventors have purified rat TPO from plasmas of thrombocytopenic rats by measuring an activity that stimulates the production of megakaryocytes from megakaryocyte progenitor cells highly purified from rat bone marrow, and have succeeded in cloning rat TPO cDNA and human TPO cDNA based on its partial amino acid sequence and in obtaining homogeneous human TPO in a large quantity by recombinant DNA techniques (H. Miyazaki et al., Exp. Hematol., vol.22, p.838 (1994)). The thus obtained human TPO has the same amino acid sequence as the above mentioned factor obtained as human Mpl ligand (see SEQ ID NO:1 described later).

The present inventors have found that the TPO of the present invention was effective for the treatment of thrombocytopenia, because thrombocytopenia inhibiting effect, thrombocytopoiesis enhancing effect and increased hematopoietic function were observed when said human TPO was administered to mice with thrombocytopenia in which bone marrow suppression has been induced by administration of a carcinostatic agent or an immunosuppressant, or by radiation or BMT.

Because of its high activity, TPO can be used in an extremely small amount, namely, it is normally administered several times a day in a dose of from 0.05 µg/kg body weight to 1 mg/kg body weight, preferably from 0.5 µg/kg body weight to 50 µg/kg body weight, as the active ingredient, depending on conditions, sex, and administration routes. Thus, it is necessary to produce TPO-containing pharmaceutical preparations using an extremely small quantity of TPO, but, in this case, it is unavoidable to cause reduction of a TPO titer due to the adsorption of TPO on containers (e.g., vials, ampoules or the like) and the loss of the TPO ingredient caused by its adsorption onto infusion assemblies (bottles or tubes) when the preparations are mixed with a infusion liquid at the time of drip infusion.

In this context, the present inventors have studied a stable TPO-containing composition which can prevent adsorption of a TPO protein having a sugar chain moiety, with the aim of preventing reduction of a TPO titer when a pharmaceutical TPO preparation is produced or when it is mixed with a transfusion liquid. As a result, it has now been found that the addition of a pharmaceutically acceptable protein, cellulose derivative, surface active agent or polyvinyl alcohol to a TPO protein having a sugar chain moiety is effective for this purpose.

### SUMMARY OF THE INVENTION

Thus, according to the present invention, there is provided a method for the prevention of the reduction of a TPO titer caused by adsorption of TPO on the wall of a container charged with a composition that contains a TPO protein having a sugar chain moiety, which comprises adding to the composition at least one pharmaceutically acceptable additive selected from the group consisting of proteins, cellulose derivatives, surface active agents (i.e., surfactants) and polyvinyl alcohol.

The present invention also provides a TPO-containing composition which comprises a TPO protein having a sugar chain moiety and at least one pharmaceutically acceptable additive selected from the group consisting of proteins, cellulose derivatives, surface active agents and polyvinyl alcohol.

### BRIEF DESCRIPTION OF THE DRAWINGS

Fig. 1 is a standard curve showing the relationship between a concentration of TPO(1-332)/CHO and a corresponding absorbance value (450 nm/650 nm), as determined by ELISA.

Fig. 2 is a graph showing the change in a % recovery of TPO(1-332)/CHO over time when human serum albumin (HSA) is added in various concentrations.

### DETAILED DESCRIPTION OF THE INVENTION

As the TPO of the present invention, a glycoprotein composed of a polypeptide having the amino acid sequence shown in SEQ ID NO:1 and a sugar chain moiety may be used, and its production method is not particularly limited, provided that the product is an isolated glycoprotein having a high purity. Also useful as the TPO of the present invention is a glycoprotein which contains a polypeptide having an amino acid sequence partially modified (by substitution, deletion, insertion and/or addition) in the amino acid sequence shown in SEQ ID NO:1, together with a sugar chain moiety, provided that it maintains the TPO activity.

In other words, a glycoprotein having a polypeptide whose amino acid sequence is substantially the amino acid sequence shown in SEQ ID NO:1 and a sugar chain moiety can also be used. The term "substantially the amino acid sequence shown in SEQ ID NO:1" as used herein means that the "amino acid sequence resulting from partial substitution, deletion, insertion and/or addition of the amino acid sequence shown in SEQ ID NO:1, provided that it maintains the TPO activity" is included in addition to the amino acid sequence shown in SEQ ID NO:1.

It has been found that recombinant human TPO proteins keep a TPO activity even if amino acid residues of the C-terminal side of the amino acid sequence shown in SEQ ID NO:1 are deleted up to the position 152, or even if amino acid residues of the N-terminal side are deleted up to the position 6. Concrete data are shown in Table 1.

**Table 1**

| Derivative | Activity |
|---|---|
| Positions 1-231 | + |
| Positions 1-211 | + |
| Positions 1-191 | + |
| Positions 1-171 | + |
| Positions 1-163 | + |
| Positions 1-157 | + |
| Positions 1-156 | + |
| Positions 1-155 | + |
| Positions 1-154 | + |
| Positions 1-153 | + |
| Positions 1-151 | + |
| Positions 1-150 | - |
| Positions 7-163 | + |
| Positions 8-163 | - |
| Positions 13-231 | - |

Thus, the TPO proteins of the present invention are glycoproteins which contains an amino acid sequence corresponding to the 7 to 151 positions of the amino acid sequence shown in SEQ ID NO:1 and has the TPO activity. More specifically, the glycoproteins include ones that individually have a sugar chain(s) and a polypeptide chain of the positions 1-231, 1-211, 1-191, 1-171, 1-163, 1-157, 1-156, 1-155, 1-154, 1-153, 1-151 or 7-163 of the amino acid sequence shown in SEQ ID NO:1.

Also included in the TPO proteins of the present invention are glycoproteins which contain a polypeptide chain having a substitution, deletion, insertion and/or addition of at least one amino acid residue inside or outside the above mentioned 7-151 sequence shown in SEQ ID NO:1, to the extent that the TPO activity is not spoiled, and has a sugar chain(s).

The TPO proteins of the present invention may include a glycoprotein in which at least the Ser¹ and Ala³ of the human TPO amino acid sequence shown in SEQ ID NO:1 are substituted by Ala and Val, respectively; a glycoprotein in which the Arg²⁵ is substituted by Asn; a glycoprotein in which the His³³ is substituted by Thr; a glycoprotein in which the Arg²⁵ is substituted by Asn and the Glu²³¹ is substituted by Lys; and glycoproteins in which the polypeptide: Thr Ser Ile Gly Tyr Pro Tyr Asp Val Pro Asp Tyr Ala Gly Val His His His His His His is added to each C-terminus of the above described glycoproteins.

Further included are glycoproteins having the deletion and/or addition of at least the following amino acid residues in the sequence shown in SEQ ID NO:1, namely, a glycoprotein in which the His³³ is deleted; a glycoprotein in which the Gly¹¹⁶ is deleted; a glycoprotein in which the Arg¹¹⁷ is deleted; a glycoprotein in which a threonine residue is inserted between the His³³ and the Pro³⁴; a glycoprotein in which an alanine residue is inserted between the His³³ and the Pro³⁴; a glycoprotein in which a glycine residue is inserted between the His³³ and the Pro³⁴; a glycoprotein in which a glycine residue is inserted between the His³³ and the Pro³⁴ and the Pro³⁸ is substituted by Ser; a glycoprotein in which an asparagine residue is inserted between the Gly¹¹⁶ and the Arg¹¹⁷; a glycoprotein in which an alanine residue is inserted between the Gly¹¹⁶ and the Arg¹¹⁷; and a glycoprotein in which a glycine residue is inserted between the Gly¹¹⁶ and the Arg¹¹⁷. Still further examples of the TPO proteins of the present invention are a glycoprotein in which at least the Leu¹²⁹ is substituted by Arg; a glycoprotein in which the His¹³³ is substituted by Arg; a glycoprotein in which the Met¹⁴³ is substituted by Arg; a glycoprotein in which the Gly⁸² is substituted by Leu; a glycoprotein in which the Gly¹⁴⁶ is substituted by Leu; a glycoprotein in which the Ser¹⁴⁸ is substituted by Pro; a glycoprotein in which the Lys⁵⁹ is substituted by Arg; and a glycoprotein in which the Gln¹¹⁵ is substituted by Arg.

Also included as the TPO proteins of the present invention are glycoproteins in which methionine and lysine residues are respectively added to the positions -2 and -1 of the human TPO protein having the amino acid sequence shown in SEQ ID NO:1 and the above described derivatives; and glycoproteins in which a methionine residue is attached at the position -1 of the human TPO protein having the amino acid sequence shown in SEQ ID NO:1 and the derivatives.

Preferably, the TPO proteins of the present invention may be obtained by isolating and purifying them from host cells transformed with a recombinant vector containing their cDNA, chromosomal DNA or chemically synthesized DNA. As the host, eucaryotic cells (e.g., from yeasts, insects or mammals) can be used. Examples of the mammalian cells include COS cell, Chinese hamster ovary (CHO) cell, X63.6.5.3. cells, C-127 cell, BHK (Baby Hamster Kidney) cell, human cells (such as HeLa cell), and so on. Examples of the yeasts include a baker's yeast (*Saccharomyces cerevisiae*), a methanol assimilating yeast (*Pichia pastoris*) and the like. Examples of the insect cells include cultured silkworm cells (e.g., Sf21 cell) and the like.

Proteins, cellulose derivatives, surface active agents, polyvinyl alcohol and the like can be exemplified as additives useful for the preparation of the stable TPO-containing composition of the present invention. Kinds of these additive agents are not particularly limited, provided that they can prevent adsorption of TPO onto containers, tubes (infusion lines) or the like, made of glass, a resin or the like, which include materials set forth below.

As the proteins, human serum albumin, gelatin, bovine serum albumin, casein, collagen, human serum globulin and the like can be used.

As the cellulose derivatives, methylcellulose, hydroxypropylcellulose, hydroxyethylcellulose and the like can be used.

Examples of useful surface active agents include polyoxyethylene hydrogenated castor oil; polyoxyethylene castor oil; polyoxyethylene sorbitan fatty acid esters such as polysorbate 80, polyoxyethylene sorbitan monolaurate (alias: polysorbate 20) and the like; polyoxyethylene polyoxypropylene glycol; sorbitan fatty acid esters such as sorbitan monooleate and the like; sucrose fatty acid esters such as sucrose monolaurate and the like; aromatic quaternary ammonium salts such as benzethonium chloride, benzalkonium chloride and the like; sodium caprylate; and sodium sulfite.

The above described additives as used in the present invention may be used in a concentration ranging from 0.001% to 10% when the TPO-containing composition is prepared in the form of an aqueous solution. Also, it is desirable to use these additives within the range of from 0.02 parts by weight to 10,000 parts by weight per part by weight of TPO protein as an active ingredient.

In addition, the TPO-containing composition of the present invention may also contain a diluent, a solubilizing agent, an antiseptic agent, an antioxidant, an excepient, an isotonicity or the like, depending on preparation purposes of the composition.

The stable TPO-containing composition of the present invention can be prepared in dosage forms such as solutions, suspensions, tablets, pills, capsules, granules or freeze-dried preparations, depending on various administration routes that include parenteral administration (e.g., injection), transpulmonary administration, transnasal administration and oral administration. The TPO and the additive as used in the present invention may be formulated such that they coexist in the same composition from the beginning, or alternatively the TPO and the additive may be separately preformulated and blended when use.

The following test examples and working examples are provided to further illustrate the present invention.

### EXAMPLES

In the following test examples, the enzyme immunoassay of TPO was carried out as follows.

### Enzyme-linked immunosorbent assay (ELISA) using anti-human TPO monoclonal antibody

An anti-human TPO monoclonal antibody (i.e., subclone L3-1-54 of L3-1) as a solid phase antibody which recognizes the human TPO HT-1 region (corresponding to the 8 - 28 amino acid sequence shown in SEQ ID NO:1) was prepared in a concentration of 20 µg/ml in 50 mM carbonate buffer (pH 9.2) and dispensed in 50 µl portions into wells of a 96-well microtiter plate (Nunc-Immuno Plate MaxiSorp™, manufactured by InterMed, Ca.No. 4-42404). The solid phase antibody was thoroughly spread on the bottom of each well while shaking the plate on a microplate mixer (ADVANTEC TS-96, manufactured by Advantech Toyo, Japan). The plate was sealed with a plate seal (manufactured by SUMILON, Ca No. MS-30020) and left at 37°C for 2 hours or at 4°C overnight to adsorb the solid phase antibody on the plate. Next, after washing with a washing buffer (20 mM Tris-HCl/0.5 M NaCl/0.1% Tween 20 (pH 7.5)), 300 µl of 4 X Block Ace (manufactured by Dainippon Pharmaceuticals, Japan, Ca No. UK-B25) was added to each well, and the plate was sealed with a plate seal and left at 37°C for 1 hour or at 4°C overnight. After washing 4 times with the washing buffer, 50 µl of a sample to be tested or a known concentration of TPO as a standard which was diluted with 10 X Block Ace, was added to each well, and the plate was shaken using a microplate mixer, sealed with a plate seal and then left at 37°C for 2 hours or at 4°C overnight. After completion of the reaction, the plate was washed 4 times with the washing buffer. Next, another anti-human TPO monoclonal antibody (i.e., subclone L4-1-31 of L4-1) as a primary antibody which recognizes the human TPO HT-2 region (corresponding to the 47-62 amino acid sequence shown in SEQ ID NO:1) was labeled with biotin, diluted to 500 ng/ml with 10 X Block Ace and dispensed in 50 µl portions into the wells, after which the plate was shaken on a microplate mixer, sealed with a plate seal and then left at 37°C for 2 hours or at 4°C overnight. After the completion of the reaction and the subsequent washing (x4) with the washing buffer, a peroxidase-labeled avidin (UltraAvidin™-Horseradish Peroxidase, manufactured by Leinco Technologies, Ca No. A106) was diluted 2,000 folds in 10 X Block Ace and dispensed in 50 µl portions into the wells. The plate was shaken on a microplate mixer, sealed with a plate seal and then subjected to 1 hour of reaction at 37°C. After the completion of the reaction and the subsequent washing (x4) with the washing buffer, 100 µl of a color forming agent prepared by adding 1/100 volumes of a substrate solution to a TMBZ color former of the color developing kit for peroxidase (manufactured by SUMILON, Ca.No. ML-1120T) was added to each well, and the plate was shaken on a microplate mixer, sealed with a plate seal and then subjected to the reaction at room temperature. After about 30 minutes, 100 µl of a reaction termination solution was added to each well, and the plate was shaken using a microplate mixer to stop the coloring reaction. Absorbances at wave lengths of 450 nm/650 nm were measured using an ELISA plate reader (THERMOmax, manufactured by Molecular Devices).

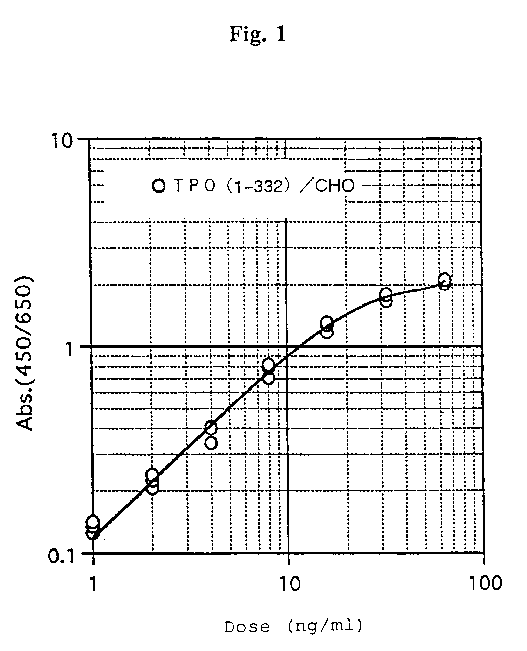

A standard curve was made based on absorbance values of known concentrations of TPO(1-332)/CHO obtained by the procedure described in Reference Example (see Fig. 1).

In this connection, the subclone (L4-1-31) of the hybridoma L4-1 and the subclone (L3-1-54) of the hybridoma L3-1 used herein, which the subclones can both produce anti-human TPO monoclonal antibodies, have been deposited on December 27, 1994 with the National Institute of Bioscience and Human Technology, Agency of Industrial Science and Technology, Ministry of International Trade and Industry, Japan, under Accession Nos. FERM BP-4956 and FERM BP-4955, respectively. The above subclones have also been deposited with the Chinese depositary authority (CCTCC) under Accession No. CCTCC-C95003 for Mouse-Mouse hybridoma L4-1-31 and Accession No. CCTCC-C95002 for Mouse-Mouse hybridoma L3-1-54, respectively.

### Test Example 1

Test samples were prepared by adding human serum albumin to 10 mM Tris buffer (pH 7.5) to a final concentration of 0.005%, 0.01%, 0.02%, 0.05%, 0.1%, 0.2% or 0.5%. As a control sample, 10 mM Tris buffer (pH 7.5) alone was used.

1.5 µl of a solution containing 3 µg of TPO obtained by the procedure described later in Reference Example was put in a glass test tube which has been charged with 1,000 µl of each sample, and was left at the room temperature. The solution was taken from the tube in 10 µl portions after 0.5 minutes, 1 hour or 2 hours, and the amount of TPO in the solution was measured by ELISA to calculate its recovery (%) based on the expected value.

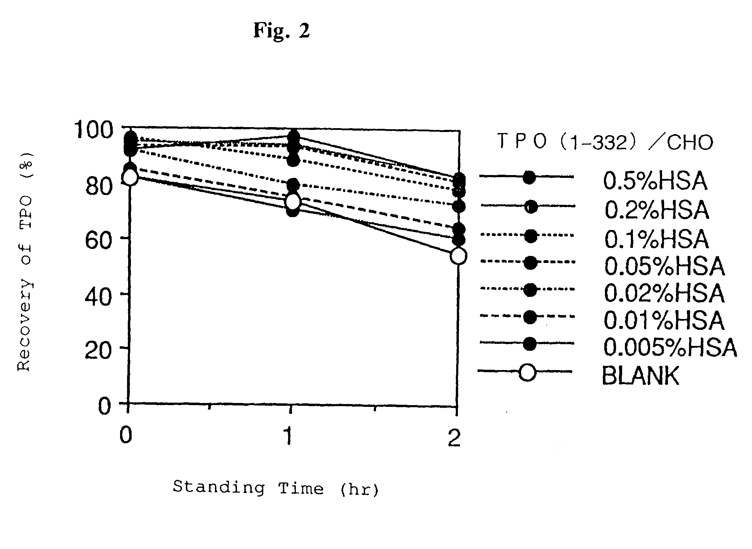

The results are shown in Fig. 2. In the drawing, BLANK indicates a case of 10 mM Tris buffer (pH 7.5) only and HSA indicates human serum albumin. It was found that human serum albumin had the adsorption preventing effect.

### Test Example 2

2.4 µl of a solution containing 3 µg of TPO [i.e., TPO(1-332)/CHO] obtained by the procedure described in Reference Example was added to a glass test tube which has been charged with 1,000 µl of a solution prepared by dissolving each of the various additives shown in Table 2 in 10 mM Tris buffer (pH 7.5) to a final concentration of 0.1%, and the recovery after 24 hours of standing was measured by ELISA. The results are shown in Table 2.

**Table 2**

| Additive (amount added, 1 mg/ml) | Recovery (%) |
|---|---|
| human serum albumin | 83.3 |
| purified gelatin (type A) | 108.8 |
| purified gelatin (type B) | 85.3 |
| methylcellulose | 87.9 |
| hydroxypropylcellulose | 86.2 |
| hydroxyethylcellulose | 73.7 |
| polyvinyl alcohol (partially saponified product) | 87.7 |
| polyoxyethylene hydrogenated castor oil 60 | 75.2 |
| polysorbate 80 | 81.5 |
| polyoxyethylene sorbitan monolaurate | 83.0 |
| polyoxyethylene(160) polyoxypropylene(30) glycol | 81.6 |
| sorbitan monooleate | 118.8 |
| sucrose monolaurate | 78.3 |
| benzethonium chloride | 120.4 |
| sodium caprylate | 69.9 |
| sodium sulfite | 75.5 |
| no addition | 59.1 |

As shown in the table, all the additives tested could prevent the adsorption of TPO when contacted to glass test tubes.

Examples of the compositions of the present invention will be set forth below.

### Example 1

An aqueous solution that contains 1,000 µg of TPO, 0.025 g of human serum albumin and 87.66 mg of sodium chloride in 10 ml of 5 mM phosphate buffer (pH 6.0) was aseptically prepared and dispensed in 1 ml portions into vials which were subsequently sealed.

### Example 2

A pharmaceutical preparation was prepared by repeating the procedure of Example 1, except that 0.1 g of purified gelatin (type B) was used instead of 0.025 g of human serum albumin.

### Example 3

An aqueous solution that contains 2,500 µg of TPO, 10 mg of polysorbate 80 and 0.5 g of sorbitol in 10 ml of 1 mM citrate buffer (pH 6.0) was aseptically prepared and dispensed in 1 ml portions into vials which were subsequently sealed.

### Example 4

An aqueous solution that contains 2,500 µg of TPO, 10 mg of polyoxyethylene hydrogenated castor oil 60 and 81.82 mg of sodium chloride in 10 ml of 10 mM Tris buffer (pH 6.5) was aseptically prepared and dispensed in 1 ml portions into vials which were subsequently sealed.

As a reference example, a process for the production of TPO as an active ingredient of the present invention is described below.

### Reference Example: Production example of TPO(1-332)

(1) CHO cells (dhfr⁻ strain; Urlaub and Chasin, Proc. Natl. Acad. Sci. USA, vol.77, p.4216, 1980) were cultured in plates (6 cm in diameter) (manufactured by Falcon) using an (α-minimum essential medium (α-MEM(-); supplemented with thymidine and hypoxanthine) containing 10% fetal calf serum, and the thus grown cells were then transformed with a plasmid pDEF202-hTPO-P1 by the calcium phosphate method (Cellphect, manufactured by Pharmacia).
   That is, 10 µg of the plasmid pDEF202-hTPO-P1 containing a cDNA insert shown in SEQ ID NO:2 was mixed with 120 µl of buffer A and 120 µl of H₂O and left for 10 minutes at the room temperature. Next, 120 µl of buffer B was added to the resulting solution, mixed again and then left for 30 minutes at room temperature. This DNA solution was added dropwise to the aforementioned plates, followed by 6 hours of cultivation in a CO₂ incubator. After removing the medium from the plates, the culture was washed twice with α-MEM(-) followed by addition of 10% dimethylsulfoxide-containing (α-MEM(-), and treated at the room temperature for 2 minutes. Next, a 10% dialyzed fetal calf serum-containing non-selection medium (α-MEM(-), supplemented with hypoxanthine and thymidine) was added to the culture which was then cultured for 2 days, after which the selection was effected using a 10% dialyzed fetal calf serum-containing selection medium (α-MEM(-), without hypoxanthine and thymidine). The selection was carried out by a procedure in which the cells from each of the 6-cm plates were treated with trypsin, divided into five new 10-cm plates or twenty 24-well plates, and then continuously cultured while exchanging the medium with the selection medium every 2 days The culture supernatant in plates or wells in which cells were grown was assayed for human TPO activity. Cells that the human TPO activity was confirmed in the culture supernatant were divided 1:15 in new plates or wells containing a 25 nM methotrexate-containing selection medium, and the cultivation was continued to effect cloning by allowing methotrexate-resistant cells to grow. In this connection, the transformation of the CHO cells can also be carried out by co-transfection of CHO cells with pHTP-1 and pMG1.
   A CHO cell strain (CHO-DUKXB11) transformed with the plasmid pDEF202-hTPO-P1 has been deposited under the terms of the Budapest Treaty on January 31, 1995 with the National Institute of Bioscience and Human Technology, Agency of Industrial Science and Technology, Ministry of International Trade and Industry, Japan, under Accession No. FERM BP-4988. The above CHO cell line has also been deposited with the Chinese depositary authority (CCTCC) under Accession No. CCTCC-C95004 (designated as CHO28/1/1/3-C6).
(2) Culture of human TPO-producing CHO cell line and purification of human TPO
   A human TPO-producing CHO cell strain (CHO28/1/1/3-C6 strain, resistant to 400 nM MTX) obtained by repeating MTX resistance was cultured in the following manner. The cells were cultured using a DMEM/F-12 medium (GIBCO) containing 400 nM MTX and 10% FCS. The grown cells were peeled off using a trypsin solution, and 1 x 10⁷ of the cells were inoculated into 200 ml of the same medium in a Falcon roller bottle (Falcon 300) and cultured at 37°C at a rotating speed of 1 rpm over 3 days. Thereafter, the culture supernatant was removed by suction, and the remaining cells were rinsed with 50 ml of PBS, suspended in 300 ml of the DMEM/F-12 medium (GIBCO) which has been supplemented with 2 µg/ml of insulin and 10 µM copper sulfate instead of 400 nM MTX and 10% FCS, and then subjected to 4 days of cultivation at 37°C at a rotating speed of 1 rpm to recover a culture supernatant (designated as Harvest-1). Subsequently, 300 ml of the above described production medium was added to the remaining cells which were then cultured at 37°C at a rotating speed of 1 rpm over 4 days to recover a culture supernatant (designated as Harvest-2).
   About 220 L of the combined serum-free CHO cell culture supernatants of Harvests-1 and -2 were passed through a 0.5 µm filter (FILTER CARTRIDGE, manufactured by Nihon Pall, Japan) and concentrated to a volume of about 5 L by ultrafiltration (CENTRASETTETM OMEGA 30k cut, manufactured by FILTRON) while simultaneously exchanging the solvent with 10 mM potassium phosphate buffer (pH 6.8). To the concentrate was added 10 µM (final concentration) of a protease inhibitor E-64 (manufactured by Peptide Institute, Japan), and the resulting solution was applied at a flow rate of 100 ml/min to an SP Sepharose FF column (11 cm in diameter and 10 cm in bed height, manufactured by Pharmacia) which has been equilibrated with 10 mM potassium phosphate buffer (pH 6.8). After washing with the equilibration buffer, elution was effected with 1,700 ml of 10 mM potassium phosphate buffer (pH 6.8) containing 0.3 M sodium chloride. The eluate was mixed with 363 g of ammonium sulfate and centrifuged at 12,000 x g for 20 minutes, and the resulting supernatant was loaded at a flow rate of 100 ml/min to a MacroPrep Methyl HIC column (6 cm in diameter and 30 cm in bed height, manufactured by Bio-Rad) which has been equilibrated with 10 mM potassium phosphate buffer (pH 6.8) containing 1.2 M ammonium sulfate. After loading, the column was washed with the equilibration buffer, and elution was effected with 700 ml of 10 mM potassium phosphate buffer (pH 6.8) containing 0.5 M ammonium sulfate. The eluate was mixed with 80 ml of propanol and loaded to a SOURCE 15 RPC column (3.5 cm in diameter and 10 cm in bed height, manufactured by Pharmacia) at a flow rate of 16 ml/min. After loading, the column was washed with 10 mM Tris buffer (pH 7.5) containing 10% propanol (designated as Development solvent A), and elution was effected with a 60-min linear gradient from Development solvent A to 10 mM Tris buffer (pH 7.5) containing 80% propanol ( designated as Development solvent B) until the propanol concentration reached 70%. 192 ml of fractions eluted at around 25 minutes after the beginning of the linear gradient were collected and divided into 2 pools, and each pool was applied to a Superdex 200 pg column (10 cm in diameter and 56 cm in bed height, manufactured by Pharmacia) which has been equilibrated with PBS, and then elution was effected at a flow rate of 40 ml/min. When the eluates were analyzed by SDS-PAGE, a protein having a molecular weight of about 65,000 to about 100,000 which was expected to be PTO was found as a single band at a retention time of around 42 to 52 minutes. A western analysis showed that this protein is TPO. When a portion of this sample was subjected to N-terminal amino acid analysis as well as to amino acid composition analysis, the results revealed that about 230 mg of TPO having the 1-322 amino acid sequence shown in SEQ ID NO:1 was obtained in a highly purified form.

## Claims

1. A thrombopoietin (TPO)-containing composition comprising a TPO protein having a sugar chain moiety and at least one pharmaceutically acceptable additive selected from the group consisting of proteins, cellulose derivatives, surface active agents and polyvinyl alcohol.

2. The TPO-containing composition according to claim 1 wherein said additive is contained in an amount of from 0.001% to 10% when the composition is in the form of an aqueous solution

3. The TPO-containing composition according to claim 1 or claim 2 wherein said protein is human serum albumin and/or gelatin.

4. The TPO-containing composition according to claim 1 or claim 2 wherein said cellulose derivative is at least one compound selected from the group consisting of methylcellulose, hydroxypropylcellulose and hydroxyethylcellulose.

5. The TPO-containing composition according to claim 1 or claim 2 wherein said surface active agent is at least one compound selected from the group consisting of polyoxyethylene hydrogenated castor oil, polyoxyethylene castor oil, a polyoxyethylene sorbitan fatty acid ester, polyoxyethylene polyoxypropylene glycol, a sorbitan fatty acid ester, a sucrose fatty acid ester, an aromatic quaternary ammonium salt, sodium caprylate and sodium sulfite.

6. A method for preventing the reduction of a TPO titer caused by adsorption of TPO on the wall of a container charged with a composition that contains a TPO protein having a sugar chain moiety, which comprises adding to the composition at least one pharmaceutically acceptable additive selected from the group consisting of proteins, cellulose derivatives, surface active agents and polyvinyl alcohol.


SEQUENCE LISTING

INFORMATION FOR SEQ ID NO:1:
(i) SEQUENCE CHARACTERISTICS:
(A) LENGTH: 332 amino acids
(B) TYPE: amino acid
(ii) MOLECULE TYPE: protein
(vi) ORIGINAL SOURCE:
(A) ORGANISM: human (Homo sapiens)
(xi) SEQUENCE DESCRIPTION: SEQ ID NO:1:
Ser Pro Ala Pro Pro Ala Cys Asp Leu Arg Val Leu Ser Lys Leu Leu
1               5                   10                  15
Arg Asp Ser His Val Leu His Ser Arg Leu Ser Gln Cys Pro Glu Val
            20                  25                  30
His Pro Leu Pro Thr Pro Val Leu Leu Pro Ala Val Asp Phe Ser Leu
        35                  40                  45
Gly Glu Trp Lys Thr Gln Met Glu Glu Thr Lys Ala Gln Asp Ile Leu
    50                  55                  60
Gly Ala Val Thr Leu Leu Leu Glu Gly Val Met Ala Ala Arg Gly Gln
65                  70                  75                  80
Leu Gly Pro Thr Cys Leu Ser Ser Leu Leu Gly Gln Leu Ser Gly Gln
                85                  90                  95
Val Arg Leu Leu Leu Gly Ala Leu Gln Ser Leu Leu Gly Thr Gln Leu
            100                 105                 110
Pro Pro Gln Gly Arg Thr Thr Ala His Lys Asp Pro Asn Ala Ile Phe
        115                 120                 125
Leu Ser Phe Gln His Leu Leu Arg Gly Lys Val Arg Phe Leu Met Leu
    130                 135                 140
Val Gly Gly Ser Thr Leu Cys Val Arg Arg Ala Pro Pro Thr Thr Ala
145                 150                 155                 160
Val Pro Ser Arg Thr Ser Leu Val Leu Thr Leu Asn Glu Leu Pro Asn
                165                 170                 175
Arg Thr Ser Gly Leu Leu Glu Thr Asn Phe Thr Ala Ser Ala Arg Thr
            180                 185                 190
Thr Gly Ser Gly Leu Leu Lys Trp Gln Gln Gly Phe Arg Ala Lys Ile
        195                 200                 205
Pro Gly Leu Leu Asn Gln Thr Ser Arg Ser Leu Asp Gln Ile Pro Gly
    210                 215                 220
Tyr Leu Asn Arg Ile His Glu Leu Leu Asn Gly Thr Arg Gly Leu Phe

225                 230                 235                 240
Pro Gly Pro Ser Arg Arg Thr Leu Gly Ala Pro Asp Ile Ser Ser Gly
                245                 250                 255
Thr Ser Asp Thr Gly Ser Leu Pro Pro Asn Leu Gln Pro Gly Tyr Ser
            260                 265                 270
Pro Ser Pro Thr His Pro Pro Thr Gly Gln Tyr Thr Leu Phe Pro Leu
        275                 280                 285
Pro Pro Thr Leu Pro Thr Pro Val Val Gln Leu His Pro Leu Leu Pro
    290                 295                 300
Asp Pro Ser Ala Pro Thr Pro Thr Pro Thr Ser Pro Leu Leu Asn Thr
305                 310                 315                 320
Ser Tyr Thr His Ser Gln Asn Leu Ser Gln Glu Gly
                325                 330

INFORMATION FOR SEQ ID NO:2:
   (i) SEQUENCE CHARACTERISTICS:
      (A) LENGTH: 1086 base pairs
      (B) TYPE: nucleic acid
      (C) STRANDEDNESS: double
      (D) TOPOLOGY: linear
   (ii) MOLECULE TYPE: cDNA to mRNA
   (vi) ORIGINAL SOURCE:
      (A) ORGANISM: human (Homo sapiens)
   (xi) SEQUENCE DESCRIPTION: SEQ ID NO:2:
GGCCAGCCAG ACACCCCGGC CAGA                                        24
ATG GAG CTG ACT GAA TTG CTC CTC GTG GTC ATG CTT CTC CTA ACT GCA      72
AGG CTA ACG CTG TCC AGC CCG GCT CCT CCT GCT TGT GAC CTC CGA GTC     120
CTC AGT AAA CTG CTT CGT GAC TCC CAT GTC CTT CAC AGC AGA CTG AGC     168
CAG TGC CCA GAG GTT CAC CCT TTG CCT ACA CCT GTC CTG CTG CCT GCT     216
GTG GAC TTT AGC TTG GGA GAA TGG AAA ACC CAG ATG GAG GAG ACC AAG     264
GCA CAG GAC ATT CTG GGA GCA GTG ACC CTT CTG CTG GAG GGA GTG ATG 312
GCA GCA CGG GGA CAA CTG GGA CCC ACT TGC CTC TCA TCC CTC CTG GGG 360
CAG CTT TCT GGA CAG GTC CGT CTC CTC CTT GGG GCC CTG CAG AGC CTC 408
CTT GGA ACC CAG CTT CCT CCA CAG GGC AGG ACC ACA GCT CAC AAG GAT 456
CCC AAT GCC ATC TTC CTG AGC TTC CAA CAC CTG CTC CGA GGA AAG GTG 504
CGT TTC CTG ATG CTT GTA GGA GGG TCC ACC CTC TGC GTC AGG CGG GCC 552
CCA CCC ACC ACA GCT GTC CCC AGC AGA ACC TCT CTA GTC CTC ACA CTG 600
AAC GAG CTC CCA AAC AGG ACT TCT GGA TTG TTG GAG ACA AAC TTC ACT 648
GCC TCA GCC AGA ACT ACT GGC TCT GGG CTT CTG AAG TGG CAG CAG GGA 696
TTC AGA GCC AAG ATT CCT GGT CTG CTG AAC CAA ACC TCC AGG TCC CTG 744
GAC CAA ATC CCC GGA TAC CTG AAC AGG ATA CAC GAA CTC TTG AAT GGA 792
ACT CGT GGA CTC TTT CCT GGA CCC TCA CGC AGG ACC CTA GGA GCC CCG 840
GAC ATT TCC TCA GGA ACA TCA GAC ACA GGC TCC CTG CCA CCC AAC CTC 888
CAG CCT GGA TAT TCT CCT TCC CCA ACC CAT CCT CCT ACT GGA CAG TAT 936
ACG CTC TTC CCT CTT CCA CCC ACC TTG CCC ACC CCT GTG GTC CAG CTC 984
CAC CCC CTG CTT CCT GAC CCT TCT GCT CCA ACG CCC ACC CCT ACC AGC 1032
CCT CTT CTA AAC ACA TCC TAC ACC CAC TCC CAG AAT CTG TCT CAG GAA 1080
GGG TAA 1086